# EUROPÄISCHE PATENTSCHRIFT

(11) **EP 3 513 260 B1**
(45) Veröffentlichungstag und Bekanntmachung des Hinweises auf die Patenterteilung: **28.10.2020**
(21) Anmeldenummer: 17790938.9
(22) Anmeldetag: 11.09.2017
(51) Int. Cl.: G05B 19/409, B25J 9/16, B25J 13/06, B29C 45/76, G16H 20/40, G16H 40/63

(54) **STEUERVORRICHTUNG UND STEUERVERFAHREN FÜR INDUSTRIELLE MASCHINEN MIT GESTEUERTEN BEWEGUNGSANTRIEBEN**
CONTROL DEVICE AND CONTROL METHOD FOR INDUSTRIAL MACHINES HAVING CONTROLLED MOVEMENT DRIVES
DISPOSITIF DE COMMANDE ET PROCÉDÉ DE COMMANDE DE MACHINES INDUSTRIELLES COMPORTANT DES MÉCANISMES DE DÉPLACEMENT COMMANDÉS

(30) Priorität: 14.09.2016 AT 508232016
(43) Veröffentlichungstag der Anmeldung: 24.07.2019
(73) Patentinhaber: KEBA AG, 4041 Linz (AT)
(72) Erfinder: STUMMER, Heinz, 4840 Vöcklabruck (AT); WIMMER, Philipp, 4693 Desselbrunn (AT)
(74) Vertreter: Burger, Hannes
(86) Internationale Anmeldenummer: PCT/AT2017/060224
(87) Internationale Veröffentlichungsnummer: WO 2018/049449

(56) Entgegenhaltungen:
- WO-A1-2005/029983
- DE-A1-102010 043 104
- JP-A- 2001 088 068
- JP-A- 2011 110 646
- US-A1- 2008 234 855
- Anonymous: "DENSO ROBOT V* SERIES H* SERIES XYC SERIES XR SERIES STARTUP HANDBOOK", DENSO Robotics, 4. Mai 2015 (2015-05-04), XP055459058, Gefunden im Internet: URL:http://web.archive.org/web/20150504073 927/densorobotics.com/download/dm/15 [gefunden am 2018-03-13]

## Beschreibung

Die Erfindung betrifft eine Steuervorrichtung für industrielle Maschinen mit gesteuerten Bewegungsantrieben für Maschinenkomponenten, insbesondere für gesteuert bewegbare Maschinenachsen, sowie ein Verfahren zum Betreiben einer elektronischen Steuervorrichtung für industrielle Maschinen mit gesteuerten Bewegungsantrieben, wie dies in den Ansprüchen 1 und 13 angegeben ist.

Aus der EP 1 403 619 B1 ist ein elektronisches Bediensystem bekannt, welches als Teil eines Fahrerinformationssystems in Kraftfahrzeugen vorgesehen ist. Zudem soll dieses Bediensystem als Anzeige- und Bedienvorrichtung in Zusammenhang mit der Steuerung von Maschinen, zum Beispiel in der industriellen Fertigung, genutzt werden können. Dieses Bediensystem umfasst eine elektronische Steuereinheit, welche über eine Recheneinheit verfügt, eine Anzeigeeinheit, welche zur Visualisierung grafischer Darstellungen geeignet ist, und eine Bedieneinheit, mit welcher manuelle Eingriffe bezüglich der Funktionalitäten des jeweiligen Systems vorgenommen werden können. Zur Veränderung von Parametern des Systems ist dabei vorgesehen, dass eine Anzeigemarke in Art eines Cursors über die Anzeigefläche der Anzeigeeinheit bewegt wird und dadurch eine zur Verfügung stehende Funktion angewählt wird. Nach entsprechender Anwahl der gewünschten Funktion durch cursorartige Verschiebung der Anzeigemarke wird ein weiteres Bedienelement betätigt, um damit Parameter verändern zu können. Dieses zweite Bedienelement ermöglicht beispielsweise eine Veränderung von Parameterwerten in Verbindung mit der zuvor über ein erstes Bedienelement ausgewählten Funktion. Die in dieser Druckschrift beschriebenen Maßnahmen sind für die Steuerung bzw. Beeinflussung von Maschinen mit Bewegungsantrieben nur bedingt geeignet. Die beschriebenen Maßnahmen eignen sich vielmehr für eine Anwendung in Verbindung mit relativ unkritischen Funktionalitäten, wie sie in Fahrerinformationssystemen von Kraftfahrzeugen vorkommen. Eine Bedienung von industriellen Maschinen mit Bewegungsantrieben wäre mit dieser vorbekannten Ausführung nur bedingt zufriedenstellend.

Die EP 1 075 979 B1 beschreibt ein Verfahren zum Betreiben einer Multifunktionsbedieneinrichtung, welche ebenso in Verbindung mit Kraftfahrzeugen vorteilhaft einsetzbar ist. Bei dieser Multifunktionsbedieneinrichtung werden Menüs und/oder Bedienfunktionen auf einer Anzeigeeinheit dargestellt und die besagten Menüs und/oder Funktionen über Tastelemente und zumindest ein Drehbetätigungselement betätigt. Zumindest eines dieser Drehbetätigungselemente ist dabei hinsichtlich seiner Drehrichtungen und Drehstellungen und/oder Raststellungen und/oder Betätigungsanschläge frei programmierbar. Diese freie Programmierung erfolgt dabei derart, dass haptische Rückmeldungen im Drehbetätigungsweg erzeugt werden, die den jeweils abgerufenen Menüs oder Funktionen zugeordnet sind. Jeder Betätigungsfunktion ist dabei ein Satz haptischer Daten zugeordnet, die dynamisch an eine Funktionsdatenänderung angepasst werden. Damit ist eine intuitive Bedienung ermöglicht, nachdem der Bedienperson haptische Rückmeldungen gegeben werden, die in Abhängigkeit der jeweiligen Menüs bzw. Funktionen automatisch angepasst werden. Eine Bedienung von industriellen Maschinen mit Bewegungsantrieben ist mit der angegebenen Vorrichtung jedoch nur bedingt praktikabel.

Das Benutzerhandbuch "DENSO ROBOT" von DENSO WAVE INCORPORATED aus den Jahren 2007-2011 beschreibt die Bedienung eines mehrachsigen Industrieroboters mit diesem zugeordneter Steuervorrichtung und einem mobilen Programmierhandgerät. Das Programmierhandgerät umfasst einen Touch-Screen, Cursor-Tasten, eine Mehrzahl von zusätzlichen Eingabetasten, sogenannte Soft-Keys bzw. funktionsvariable Funktionstasten und ein endlos verdrehbares Drehsteller-Eingabeelement in Form eines sogenannten Jog-dial. Zur Auswahl von diversen Parametern, welche am Touch-Screen angezeigt werden, sind die Cursor-Tasten oder das Jog-dial zu verwenden, zur Veränderung von Parameterwerten ist entweder das Jog-dial oder eine Funktionstaste zu betätigen und nachfolgend ist eine Eingabetaste in Art einer baulich diskret ausgeführten OK-Taste oder in Art einer virtuellen OK-Eingabetaste am Touch-Screen zu drücken. Das Hin- und Herspringen zwischen Touch-Screen-Eingaben, Soft-Keys, Cursor-Eingabetasten, sonstigen Eingabetaste und dem Jog-dial erfordert höchste Konzentration für den Bediener und kann eine potentielle Fehlerquelle darstellen.

Die WO 2005/029983 A1 offenbart eine elektronische Bedieneinheit für eine Maschine, welche Bedieneinheit eine Bedienvorrichtung mit einem berührungsempfindlichen Bildschirm zur Darstellung und Betätigung einer grafischen Bedienoberfläche aufweist und eine Bedienoberflächen-Steuerungseinrichtung umfasst, die zur Steuerung der grafischen Bedienoberfläche eingerichtet ist. Die Bedienvorrichtung umfasst ein elektromechanisches Drehsteller-Betätigungselement, welches während dem Betätigungsvorgang eine Führung für die Hand des Bedieners darstellt und dadurch unerwünschte Fehlbedienungen bei Blindbedienung der Bedienvorrichtung hintan halten soll. Dem elektromechanischen Drehsteller-Betätigungselement ist mittels der grafischen Bedienoberfläche eine Mehrzahl von Bedientätigkeiten zuordenbar, sodass infolge der Betätigung des Drehsteller-Betätigungselements eine jeweils zugeordnete Bedientätigkeit ausführbar ist.

Die DE 10 2010 043 104 A1 beschreibt eine elektronische Bedienvorrichtung zur Bedienung von mindestens einem elektrischen Gerät, welches wenigstens einen Funktionsparameter aufweist, insbesondere für ein Audio-Mischpult für den Einsatz bei Radio- und/oder Fernsehübertragungen und/oder in Film- und/oder Tonstudios. Diese Bedienvorrichtung umfasst einen berührungs- oder annäherungsempfindlichen Bildschirm, welcher nach dem resistiven oder kapazitiven Sensorprinzip arbeitet, sowie ein elektromechanisches Bedienelement in Form eines Drehreglers oder Schiebereglers unmittelbar neben dem berührungs- oder annäherungsempfindlichen Bildschirm. Die Bedienvorrichtung ist dazu ausgebildet, eine Kontrolle über den/die Funktionsparameter durch Anwahl von wenigstens einem Symbol auf dem berührungs- oder annäherungsempfindlichen Bildschirm an das elektromechanische Bedienelement zuzuweisen. Diese Zuweisung der Kontrolle über den zumindest einen Funktionsparameter an das elektromechanische Bedienelement kann dabei nur während der Anwahl oder zeitgleich mit der Anwahl des zumindest einen Symbols aktiv sein. Insbesondere nähert der Benutzer seine Hand an das Symbol bzw. an die Symbole an und/oder der Benutzer berührt dieses Symbol bzw. diese Symbole, wodurch er dem elektromechanischen Bedienelement die Kontrolle über den/die Funktionsparameter während der Annäherung und/oder Berührung zuweist. Vorzugsweise nähert der Benutzer die erste Hand an das Symbol bzw. an die Symbole an und/oder berührt dieses bzw. diese Symbole, wobei er die erste Hand über oder auf dem Symbol bzw. den Symbolen behält und währenddessen mit der zweiten Hand den bzw. die Funktionsparameter am elektromechanischen Bedienelement einstellt, steuert und/oder regelt. Insbesondere behält der die erste Hand solange auf oder über dem Symbol bzw. den Symbolen, wie er dem elektromechanischen Bedienelement die Kontrolle über den bzw. die Funktionsparameter zuweist. Alternativ oder ergänzend kann vorgesehen sein, dass die Zuweisung der Kontrolle über den zumindest einen Funktionsparameter an das elektromechanische Bedienelement nach der Anwahl des zumindest einen Symbols daueraktiviert ist.

Die US 2008/0234855 A1 beschreibt ein Handbediengerät für Werkzeugmaschinen, welches ein Display, mehrere diskret aufgebaute Eingabetasten und ein Drehsteller-Betätigungselement mit zumindest zwei turmförmig aufgebauten, koaxial zueinander angeordneten Betätigungsorganen umfasst. Den jeweiligen Betätigungsorganen sind dabei Mechanismen zugeordnet, mit welchen eine selbsttätige Rückstellung in definierte Ruhe- bzw. Ausgangsstellungen ermöglicht werden soll.

Die JP 2001-088068 A und JP 2011-110646 A betreffen weitere mobile Handbediengeräte zur Überwachung sowie zur softwarebasierten, menügeführten Steuerung und/oder Programmierung von Industrierobotern.

Aufgabe der vorliegenden Erfindung war es, die Nachteile des Standes der Technik zu überwinden und eine Vorrichtung und ein Verfahren zur Verfügung zu stellen, mittels derer ein Benutzer in der Lage ist, eine möglichst praktikable Bedienung von industriellen Maschinen mit Bewegungsantrieben vorzunehmen.

Insbesondere liegt eine Aufgabe der vorliegenden Erfindung darin, die Bedien- bzw. Programmierbarkeit von Maschinen mit gesteuert verfahrbaren Komponenten zu verbessern.

Diese Aufgabenstellung wird durch eine Vorrichtung und ein Verfahren gemäß den Ansprüchen gelöst.

Demnach ist eine Steuervorrichtung für industrielle Maschinen mit gesteuerten Bewegungsantrieben für Maschinenkomponenten vorgesehen, welche Steuervorrichtung zumindest eine Mensch-Maschine-Schnittstelle, insbesondere steuerungstechnische Ein- und Ausgabemittel, umfasst. Dabei ist wenigstens ein Bedienelement zur manuellen Beeinflussung oder Vorgabe von Verstellbewegungen von zumindest einer der Maschinenkomponenten, beispielsweise in Art von gesteuert verstellbaren Maschinenachsen, ausgeführt. Ein berührungssensitiver Bildschirm, insbesondere eine sogenannter Touch-Screen, ist zur Anzeige und bedarfsweisen Veränderung steuerungsrelevanter Parameter durch eine Bedienperson vorgesehen. Die entsprechende Steuervorrichtung zeichnet sich dadurch aus, dass zumindest ein Bedienelement als Drehsteller-Bedienelement mit einem endlos rotierbaren, insbesondere anschlaglos verdrehbaren Betätigungsorgan ausgeführt ist, und dass das Drehsteller-Bedienelement und der berührungssensitive Bildschirm mit einer elektronischen Auswerte- und Steuervorrichtung steuerungstechnisch verbunden sind. Die Auswerte- und Steuervorrichtung ist unter anderem dazu eingerichtet, eine Auswahl eines Parameters seitens einer Bedienperson durch Berührung eines gewünschten Parameter-Feldes zu erkennen. Diese Auswahl erfolgt vorteilhaft durch eine stetige Berührung des jeweiligen Parameter-Feldes mit einem Finger der Bedienperson. Zur Veränderung von Inhalten des Parameter-Feldes bzw. zur Einstellung eines gewünschten Parameterwertes für das jeweilige Parameter-Feld ist das Betätigungsorgan des Drehsteller-Betätigungselementes von einer Bedienperson um einen entsprechenden Drehwinkel zu verdrehen, wobei die Auswerte- und Steuervorrichtung das Ausmaß der Drehbewegung erfasst und zur proportionalen Veränderung der Inhalte des Parameter-Feldes oder des ausgewählten Parameterwertes eingerichtet ist.

Entsprechend einer zweckmäßigen Maßnahme ist vorgesehen, dass der Finger der Bedienperson auf dem jeweiligen Parameter-Feld verbleibt, während die Veränderung des Parameters durch eine Drehbetätigung des Betätigungsorganes erfolgt. Demnach ist vorgesehen, dass die Berührung des jeweiligen Parameter-Feldes und die Drehbetätigung des Betätigungsorganes gleichzeitig zu erfolgen hat, um eine Veränderung bzw. Einstellung der jeweiligen Inhalte des Parameter-Feldes bzw. des jeweiligen Parameterwertes zu bewirken.

Durch die angegebenen Maßnahmen ist eine verbesserte manuelle Bedienung bzw. Kontrolle von Bewegungsantrieben bzw. von damit bewegten Maschinenkomponenten ermöglicht. Insbesondere ist dadurch für eine Bedienperson eine komfortable und zu gleich rasch durchführbare Veränderung bzw. Einstellung von Parametern einer zumindest teilweise automatisiert gesteuerten Maschine ermöglicht. Das entsprechende Bedienkonzept kann dabei bei einer Vielzahl von Produktionsmaschinen, insbesondere bei Spritzgießmaschinen, Werkzeugmaschinen und dergleichen, vorteilhaft zur Anwendung kommen. Das endlos verdrehbare Betätigungsorgan des Drehsteller-Bedienelementes bietet dabei den Vorteil, dass eine rasche Veränderung der jeweiligen Parameter ermöglicht ist und insbesondere keine Rückstell-Handlungen erforderlich sind, wenn von der Bedienperson der Parameterwert eines anderen bzw. nächsten Parameter-Feldes zu verändern ist. Insbesondere kann jeder Ruhezustand des Betätigungsorgans als Start- bzw. Nullpunkt für eine weitere Veränderung oder für eine Veränderung eines anderen Parameters genutzt werden. Von Vorteil ist in diesem Zusammenhang auch, dass das Drehsteller-Bedienelement als Eingabeelement für eine Mehrzahl bzw. Vielzahl von Parametern genutzt werden kann.

Ein Vorteil der angegebenen Maßnahmen liegt auch darin, dass eine Bedienperson deutlich seltener einen Blick- bzw. Fokuswechsel zwischen dem ausgewählten Parameter und der Eingabestelle durchführen muss. Dies insbesondere deshalb, weil der Blick der Bedienperson auf den berührungssensitiven Bildschirm gerichtet sein kann, während eine Hand der Bedienperson das Betätigungsorgan des Drehsteller-Bedienelementes ergreift und betätigt. Auch die Wahrscheinlichkeit von Falscheingaben wird dadurch wesentlich reduziert, nachdem sich die Bedienperson verstärkt auf die Veränderung der jeweiligen Parameter in Abhängigkeit der Verdrehung des Betätigungsorganes konzentrieren kann. Ständige Blick- bzw. Fokuswechsel zwischen einer Anzeigestelle des Parameters und einer bislang üblichen. Software- oder hardwaretechnisch ausgeführten Eingabetastatur bzw. einem Eingabefeld mit einer Vielzahl von Tasten sind somit vermieden bzw. erübrigt. Bislang konnte eine Bedienperson zwar Blindeingaben vornehmen, bei welchen die Bedienperson nicht auf die Eingabetastatur bzw. auf das Tastenfeld geblickt hatte, dabei ist jedoch die Gefahr von fehlerhaften Eingaben relativ hoch gewesen bzw. war die entsprechende Eingabezeit relativ lange. Dieses Problem wird durch die erfindungsgemäßen Maßnahmen weitgehend gelöst.

Ein weiterer besonderer Vorteil der erfindungsgemäßen Maßnahmen liegt darin, dass eine Bedienperson dazu angehalten ist, beide Hände zur Einstellung bzw. Veränderung von Parametern zu verwenden. Dadurch können entsprechende Bedienhandlungen besonders rasch und auch fehlervermeidend ausgeführt werden. Zudem ist der Bedienkomfort relativ hoch. Insbesondere hat die Bedienperson mit den Fingern der ersten Hand das jeweilige Parameter-Feld auszuwählen und hat die Bedienperson sodann mit der zweiten Hand via entsprechende Drehbetätigung des hardwaremäßig konzipierten bzw. baulich ausgeführten Betätigungsorganes des Drehsteller-Bedienelementes die jeweiligen Parameterveränderungen durchzuführen. Dies ermöglicht besonders kurze Eingabezeiten und auch eine relativ zielgenaue Erreichung der jeweils gewünschten Sollwerte. Darüber hinaus sind Parameter- bzw. Werteveränderungen in Verbindung mit einem endlos rotierbaren Betätigungsorgan für eine Bedienperson deutlich praktikabler umzusetzen, als dies durch eine Betätigung von Drucktasten bzw. Kippschaltern der Fall wäre.

Von Vorteil bei den angegebenen Maßnahmen gemäß Anspruch 1 ist auch, dass dadurch ein rascher und unmissverständlicher Abschluss der entsprechenden Parameterveränderungen zur Verfügung steht. Die jeweilige Systematik ist dabei von einer Bedienperson rasch bzw. einfach erlernbar und sind bereits nach kurzer Einarbeitungszeit entsprechende Maschinenbedienungen bzw. Parametereinstellungen sicher und fehlerfrei ausführbar. Darüber hinaus sind dadurch keine gesonderten Bedienhandlungen an einer anderen Position der Mensch-Maschinenschnittstelle bzw. des Bedienpanels erforderlich, sodass weitläufige Hand- bzw. Fingerbewegungen erübrigt sind. Neben einem geringen Ausmaß an Hand- bzw. Fingerbewegungen kann dadurch auch die Anzahl von erforderlichen Hand- bzw. Fingerbewegungen relativ gering gehalten werden. Letztendlich ist der entsprechende Betätigungs- bzw. Übernahmeprozess zur Quittierung der vorgenommenen Einstellungen des Parameters besonders komfortabel und rasch durchführbar.

Von Vorteil sind aber auch die Maßnahmen gemäß Anspruch 2, da dadurch ein Abbruch der jeweiligen Einstellvorgänge intuitiv und unmissverständlich vorgenommen werden kann. Nachdem die Bedienperson im Zuge der Veränderung der Parametereinstellungen ohnehin den Finger auf dem jeweiligen Parameter-Feld abgestützt hat und aufgrund eines Abbruch-Wunsches den Finger sodann streichend bzw. gleitend aus dem Parameter-Feld wegbewegt, ist für die Auswerte- und Steuervorrichtung klar erkennbar, dass es sich hierbei um einen Abbruch-Wunsch seitens der Bedienperson handelt. Die entsprechende Bedienhandlung ist ebenso rasch und komfortabel durchführbar.

Praktikabel ist auch eine Ausführungsform nach Anspruch 3, da dadurch mehrere Parameter bzw. mehrere Parameter-Felder simultan einer Wertveränderung unterzogen werden können. Hierzu kann von der Bedienperson in einfacher Art und Weise eine Gruppe von Parametern, beispielsweise in Art von Temperatur-Parametern, mit mehreren Fingern einer Hand selektiert werden und kann sodann durch einfaches Verdrehen des Betätigungsorgans mit der anderen Hand eine gemeinsame Veränderung der Parameterwerte in diesen Parameter-Feldern ausgeführt werden. Auch dadurch ist ein hoher Benutzerkomfort erzielbar und sind zudem die entsprechenden Einstellprozesse rasch durchführbar.

Zweckmäßig ist auch eine Ausführungsform nach Anspruch 4, da dadurch auch große Werte- bzw. Einstellbereiche rasch überstrichen bzw. abgedeckt werden können. Insbesondere ist es dadurch ermöglicht, von einer raschen Werteveränderung zu einer Werteveränderung in kleinen Schritten - und umgekehrt - mühelos umzuschalten und so den jeweiligen Einstellprozess für eine Bedienperson komfortabel und mühelos zu gestalten.

In diesem Zusammenhang sind die auch die Maßnahmen gemäß Anspruch 5 und/oder 6 von Vorteil, da dadurch ein besonders intuitives Bedienkonzept vorliegt, welches einer Bedienperson die entsprechenden Bedienhandlungen leicht verständlich machen kann. Insbesondere ist dadurch eine hohe Intuitivität in Bezug auf eine beabsichtige Veränderung der Einstellgeschwindigkeit der Parameterwerte gewährleistet.

Von besonderer Zweckmäßigkeit ist eine Ausführungsform gemäß Anspruch 7, da dadurch ausgehend von der Steuervorrichtung in Richtung zur Bedienperson ein Feedback-Kanal aufgebaut werden kann, welcher die Benutzerfreundlichkeit des Steuerungssystemes bzw. der Maschinensteuerung wesentlich erhöhen kann. Insbesondere kann dadurch eine verbesserte Interaktion zwischen der Bedienperson und der anzusteuernden Maschine erreicht werden und ist zudem eine relativ sichere, fehlervermeidende und zugleich rasche Bedienhandlung erzielbar. Durch die Implementierung einer haptischen Signalisierung bzw. durch Ausnutzung des taktilen Wahrnehmungsvermögens einer Bedienperson können die jeweiligen Bewegungsabläufe bzw. Parameter-Einstellvorgänge geordnet und relativ prozesssicher initiiert werden.

Praktikables Feedback kann durch die Maßnahmen gemäß Anspruch 8 erzielt werden. Insbesondere wird dadurch erreicht, dass der Bedienperson eine aktuell vorliegende Veränderungsschrittweite bzw. ein entsprechendes Veränderungsausmaß der jeweiligen Parameter haptisch vermittelt wird. Dabei wird das taktile Wahrnehmungsvermögen einer Bedienperson ausgenutzt, um eine zielgenaue und rasche Veränderbarkeit von Parameterwerten zu erreichen. Beispielsweise kann dadurch ein allzu rasches, sogenanntes "Übersteuern" vermieden werden bzw. kann anderseits einer Bedienperson auch vermittelt werden, dass die entsprechenden Einstellvorgänge rascher ausgeführt werden könnten, als diese aktuell durch die Bedienperson vorgenommen werden.

Durch die Maßnahmen gemäß Anspruch 9 wird eine hohe Intuitivität in Bezug auf das Vorliegen von hohen bzw. niedrigen Veränderungsschrittweiten bzw. Veränderungsgeschwindigkeiten gewährleistet. Dies begünstigt eine rasche und möglichst fehlervermeidende Einstellung von diversen Maschinen-Parametern.

Von hoher Zweckmäßigkeit sind auch die Maßnahmen gemäß Anspruch 10, da dadurch einer Bedienperson eindeutig signalisiert werden kann, dass die jeweiligen Bereichs- bzw. Wertegrenzen erreicht sind. Eine entsprechende haptische Signalisierung bietet dabei den besonderen Vorteil, dass die Bedienperson ihren Blick nicht auf die Parameter-Felder richten muss bzw. nicht auf die Anzeige wenden muss, um erkennen zu können, dass eine Bereichs- bzw. Wertegrenze erreicht wurde und eine weitere Veränderung des Parameterwertes unzulässig oder ungünstig ist. Insbesondere kann dadurch die Bedienperson ihren Blick auf andere Abschnitte bzw. auf die anzusteuernde Maschine richten und muss nicht das Anzeigefeld im Auge behalten. Die Erreichung eines Grenzwertes wird einfach und praktikabel haptisch signalisiert und ist die entsprechende taktile Wahrnehmung für eine Bedienperson überaus eindeutig und unmissverständlich. Ein Vorteil des grundsätzlich endlos verdrehbaren Drehsteller-Bedienelementes liegt dabei darin, dass keine fix bzw. permanent vorliegenden Endanschläge vorhanden sind, sondern dass die jeweiligen Drehbeweglichkeitsgrenzen des Betätigungsorgans automatisiert bzw. gesteuert eingestellt werden können. Diese Steuerung erfolgt dabei in praktikabler Art und Weise in Abhängigkeit des jeweiligen Parameter-Feldes. Folglich ist eine gesteuerte bzw. automatisierte Anpassung an eine Vielzahl von unterschiedlichen Wertebereichen bzw. Wertegrenzen möglich. Somit ist die anspruchsgemäße Steuervorrichtung überaus flexibel hinsichtlich unterschiedlicher bzw. variabler Wertebereiche bzw. Bereichsgrenzen.

Zweckmäßig sind auch die Maßnahmen gemäß Anspruch 11, da dadurch einer Bedienperson die jeweiligen Teilabschnitte innerhalb eines verfügbaren Wertebereiches haptisch signalisiert werden können. Die jeweiligen Drehwiderstands-Erhöhungen sind dabei von der Bedienperson leicht überwindbar. Insbesondere können diese impulsartig umgesetzt sein. Auch dadurch kann der jeweilige Einstellvorgang für eine Bedienperson möglichst komfortabel gestaltet werden und ist zudem eine besonders rasche Auffindung des jeweils gewünschten Sollwertes im jeweiligen Parameter-Feld ermöglicht. Von Vorteil ist dabei auch, dass durch das gesteuerte Drehwiderstands-Generierungsmittel eine hochflexible Anpassung an die jeweiligen Erfordernisse bzw. Bedürfnisse in Bezug auf die verschiedenen Parameter-Felder ermöglicht ist.

Vorteilhaft sind auch die Maßnahmen gemäß Anspruch 12, da dadurch der Bedienperson haptisch signalisiert wird, welche Fortschritte in Bezug auf die Parameterveränderungen vorliegen. Ein besonderer Vorteil der anspruchsgemäßen Maßnahmen liegt darin, dass die Auswerte- und Steuervorrichtung und das davon angesteuerte Drehwiderstand-Generierungsmittel eine flexible bzw. variable Erzeugung von Raststufen ermöglichen. Insbesondere kann dadurch eine automatisierte Anpassung an die jeweiligen Zustände bzw. Maschinen-Parameter vorgenommen werden. Auch dadurch kann der Bedienungskomfort und die Zügigkeit hinsichtlich der Einstellung der jeweiligen Soll- bzw. Wunschwerte gesteigert werden.

Die Aufgabe der Erfindung wird weiters durch das anspruchsgemäße Verfahren zum Betreiben einer elektronischen Steuervorrichtung für industrielle Maschinen gelöst. Die damit erzielbaren vorteilhaften Wirkungen und technischen Effekte sind den vorstehenden Ausführungen und den nachfolgenden Beschreibungsteilen zu entnehmen.

Zum besseren Verständnis der Erfindung wird diese anhand der nachfolgenden Figuren näher erläutert.

Es zeigen jeweils in stark vereinfachter, schematischer Darstellung:
- Fig. 1: eine aus mehreren Maschinen, insbesondere Industrierobotern, gebildete technische Anlage und ein dabei eingesetztes, elektronisches Steuerungssystem, welches Steuerungssystem mehrere Steuervorrichtungen und eine Mensch-Maschine-Schnittstelle in Art eines tragbaren Handbediengerätes umfasst;
- Fig. 2: eine Produktionsmaschine, insbesondere eine Spritzgießmaschine, welche eine elektronische Steuervorrichtung und eine daran angebundene Mensch-Maschine-Schnittstelle in Art eines stationären Bedienpanels umfasst;
- Fig. 3: das Bedienpanel der Produktionsmaschine nach Fig. 2;
- Fig. 4: eine Steuervorrichtung für industrielle Maschinen mit einem Drehsteller-Bedien-element, welches zur Einstellung oder Veränderung von steuerungsrelevanten Maschinen-Parametern vorgesehen ist.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind diese Lageangaben bei einer Lageänderung sinngemäß auf die neue Lage zu übertragen.

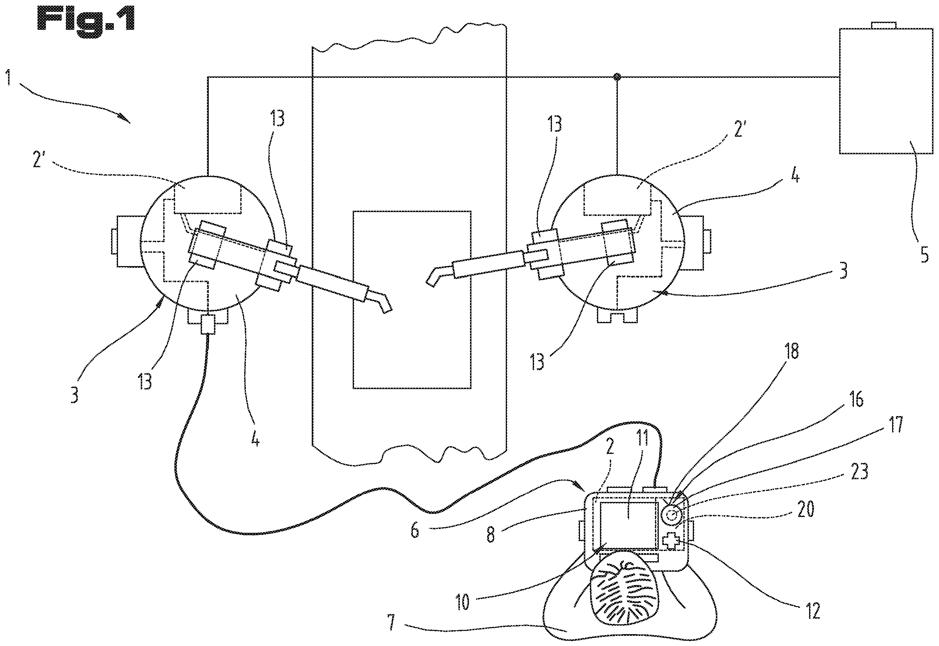

In den Fig. 1 bis 3 sind Ausführungsbeispiele von elektrotechnischen bzw. elektronischen Steuerungssystemen 1 gezeigt, die für die Automatisierung bzw. Steuerung von industriellen Anlagen eingesetzt werden können. Eine solche industrielle Anlage bzw. dessen Steuerungssystem 1 umfasst wenigstens eine elektronische Steuervorrichtung 2, 2' bzw. kann auch eine Mehrzahl von verteilt angeordneten elektronischen Steuervorrichtungen 2, 2' vorgesehen sein. Eine entsprechende Anlage umfasst zumindest eine Maschine 3 bzw. eine Mehrzahl von gegebenenfalls interagierenden Maschinen 3 bzw. Maschinenkomponenten. Die zumindest eine elektronische Steuervorrichtung 2, 2' ist vorzugsweise softwaregesteuert ausgeführt und dient primär dazu, die jeweiligen Steuerungsfunktionen der jeweiligen industriellen Maschine 3 umzusetzen bzw. die Abläufe der Maschine 3 überwachen, beeinflussen und/oder programmieren zu können.

Entsprechend der Ausführung nach Fig. 1 ist eine solche industrielle Maschine 3 durch wenigstens einen Industrieroboter 4 gebildet. Ein solcher Industrieroboter 4 kann Bestandteil einer Montage- bzw. Fertigungsanlage sein. Durch eine datentechnische Vernetzung der jeweiligen Steuervorrichtungen 2, 2' kann vorgesehen sein, dass die Industrieroboter 4 steuerungstechnisch interagieren können. Eine solche daten- bzw. steuerungstechnische Vernetzung zwischen mehreren Industrierobotern 4 kann auch einen zentralen Leitrechner 5 umfassen. Hinsichtlich einer zentralen, dezentralen, hierarchischen oder anderweitig aufgebauten Steuerungsarchitektur und Vernetzung sind dabei vielfältigste Ausführungsformen denkbar, welche entsprechend den jeweiligen Erfordernissen gewählt werden können.

Zumindest einer Steuervorrichtung 2' in zumindest einer Maschine 3 ist dabei zumindest eine Mensch-Maschine-Schnittstelle 6 (HMI) zugeordnet bzw. zuordenbar. Mittels dieser Mensch-Maschine-Schnittstelle 6 sind steuerungsrelevante Interaktionen zwischen einer Bedienperson 7 und der jeweiligen Maschine 3 ermöglicht.

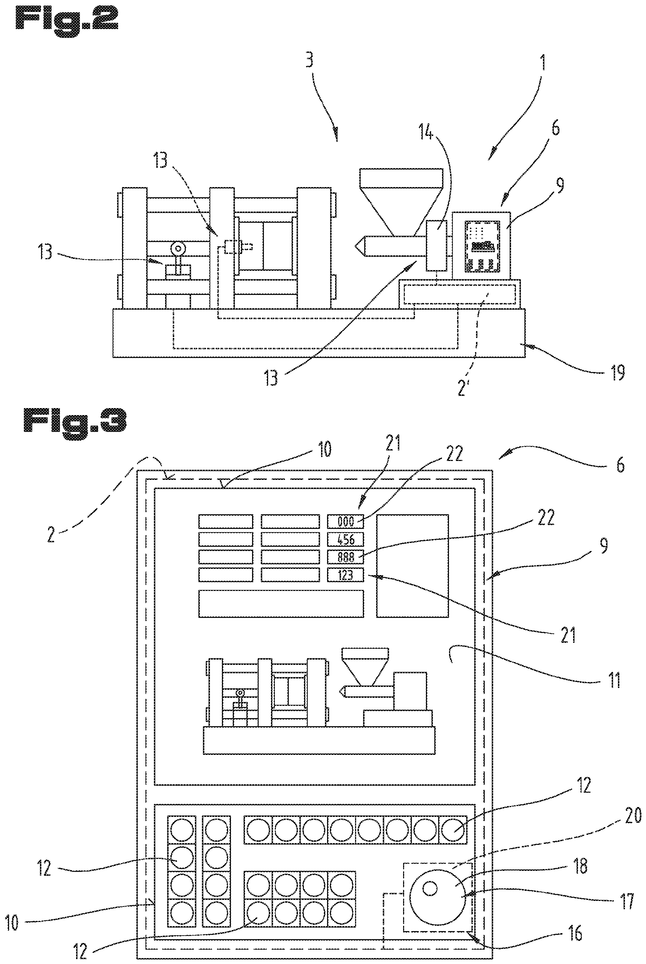

Beim Ausführungsbeispiel gemäß Fig. 1 ist die steuerungstechnische Mensch-Maschine-Schnittstelle 6 durch ein mobiles bzw. tragbares Handbediengerät 8 gebildet. Bei der Ausführungsform nach Fig. 2 ist die Mensch-Maschine-Schnittstelle 6 durch ein stationäres Bedienpanel 9 definiert. Die jeweiligen Mensch-Maschine-Schnittstellen 6 können somit auch als Bedienerschnittstellen bezeichnet werden.

Ein gattungsgemäßes Handbediengerät 8 bzw. Bedienpanel 9 weist wenigstens eine Eingabevorrichtung 10, insbesondere einen berührungssensitiven Bildschirm 11, Eingabetasten 12, Schalter, oder sonstige elektrische oder elektromechanische Eingabemittel auf. Zudem können visuell und/oder akustisch erfassbare Ausgabemittel vorgesehen sein. Bei einem gattungsgemäßen Handbediengerät 8 bzw. Bedienpanel 9 können insbesondere der zuvor erwähnte berührungssensitive Bildschirm 11, sowie Leuchtelemente bzw. Signalisierungslampen zur Anzeige von systemrelevanten Daten bzw. Zuständen vorgesehen sein. Der Funktionsumfang und die Ausführungsform der jeweiligen Eingabevorrichtung bzw. der jeweiligen Ausgabevorrichtung hängen dabei stark von der jeweiligen Anwendung, insbesondere von der technischen Komplexität der zu steuernden Maschine 3 bzw. Anlage ab. Wesentlich ist dabei, dass die Bedienperson 7 mittels der Eingabevorrichtung 10 und einer geeigneten Ausgabevorrichtung, insbesondere mittels dem zuvor genannten berührungssensitiven Bildschirm 11, die benötigten steuerungstechnischen Abläufe kontrollieren bzw. überwachen, beeinflussen und/oder programmieren kann.

Die in der Mensch-Maschine-Schnittstelle 6, insbesondere im Handbediengerät 8 bzw. im Bedienpanel 9 ausgeführte Steuervorrichtung 2 und die einer Maschine 3 zugeordnete Steuervorrichtung 2' können dabei über kabelgebundene und/oder drahtlos ausgeführte Kommunikationsschnittstellen in datentechnischer bzw. steuerungstechnischer Wechselwirkung stehen.

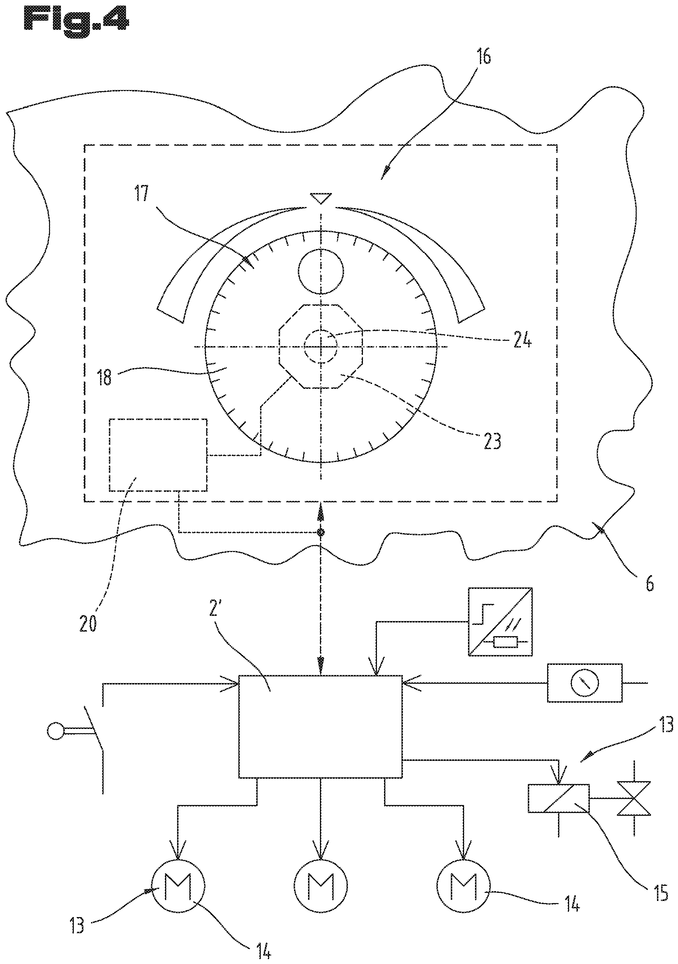

Wie an sich bekannt, sind zur Automatisierung der jeweiligen Maschinen 3 steuerbare, insbesondere wenigstens aktivier- und deaktivierbare Bewegungsantriebe 13 vorgesehen, welche mit der jeweiligen Steuervorrichtung 2' leitungsverbunden sind. Häufig sind solche Bewegungsantriebe 13 auch hinsichtlich ihrer Antriebsgeschwindigkeit und/oder Antriebsleistung bzw. Antriebskraft bedarfsabhängig einstell- bzw. veränderbar. Derartige Bewegungsantriebe 13 können, wie in Fig. 2 bzw. Fig. 4 veranschaulicht, durch Motoren 14, durch Hydraulikzylinder, durch Proportional-Magnetventile 15, oder durch sonstige Elemente zur aktiven bzw. steuerbaren Bewegung von Maschinenkomponenten gebildet sein. Unter den entsprechenden Bewegungsantrieben 13 sind auch Aktoren zu verstehen, mit welchen eine Verstellbewegung einer Maschinenkomponente erzeugt bzw. eingeleitet werden kann. Ein solcher Bewegungsantrieb 13 und die jeweilige Maschinenkomponente können dabei auch als Maschinenachse bezeichnet werden. Unter einer steuerbaren Maschinenkomponente bzw. Maschinenachse kann zum Bespiel ein Gelenksarm eines Industrieroboters 4, eine Vorschubeinheit, ein Bearbeitungsaggregat einer Werkzeugmaschine, ein Stellantrieb einer Produktionsmaschine, und dergleichen verstanden werden. An eine solche Maschinen-Steuervorrichtung 2' können typischerweise auch eine Mehrzahl von Sensoren, Endschalter und/oder Geber angeschlossen sein, wie dies allgemein bekannt ist und in Fig. 4 beispielhaft gezeigt ist. Dadurch können Bewegungs- bzw. Funktionsabläufe der jeweiligen Maschine vollautomatisch oder zumindest teilautomatisch ausgeführt werden bzw. automatisiert überwacht werden.

Zur manuellen Beeinflussung bzw. zur Programmierung der jeweiligen Bewegungsantriebe 13 bzw. Maschinenkomponenten ist an der jeweiligen Mensch-Maschine-Schnittstelle 6 zumindest ein Schalt- bzw. Bedienelement zur manuellen Beeinflussung oder Vorgabe von Verstellbewegungen von zumindest einer der Maschinenkomponenten bzw. Maschinenachsen vorgesehen. Diese manuelle Beeinflussung oder Vorgabe von Verstellbewegungen durch eine Bedienperson 7 umfasst vorzugsweise die Möglichkeit einer Geschwindigkeits- und/oder Leistungsveränderung des anzusteuernden bzw. selektiv ansteuerbaren Bewegungsantriebes 13. Zudem können Schalt- bzw. Bedienelemente, insbesondere Tast- oder Schaltelemente, ausgeführt sein, welche zur Aktivierung und Deaktivierung eines ausgewählten bzw. ansteuerbaren Bewegungsantriebes 13 vorgesehen sind.

Zumindest ein Bedienelement 16 an der Mensch-Maschine-Schnittstelle 6 ist dabei als Drehsteller-Bedienelement 17 mit einem endlos bzw. anschlaglos verdrehbaren Betätigungsorgan 18 ausgeführt. Endlose Verdrehbarkeit bedeutet dabei, dass das Drehsteller-Bedienelement 17 bzw. dessen Betätigungsorgan 18 derart ausgeführt ist, dass es keine mechanischen Endanschläge bzw. keine dauerhafte Begrenzung hinsichtlich der Drehbeweglichkeit des Betätigungsorganes 18 gibt. Dies im Unterschied zu einem typischen Potentiometer bzw. einstellbaren, ohmschen Widerstand, bei welchem ein Verdreh- bzw. Einstellbereich von üblicherweise etwa 270° gegeben ist. Das anspruchsgemäße Drehsteller-Bedienelement 17 ist vielmehr mit einem sogenannten Override-Potentiometer vergleichbar bzw. kann das Drehsteller-Bedienelement 17 als endlos drehbeweglicher Inkrementalgeber ausgeführt sein. Wesentlich ist, dass das Drehsteller-Bedienelement 17 eine endlose Rotierbarkeit seines beispielsweise scheiben- oder radförmigen Betätigungsorganes 18 bzw. eine damit einhergehende, unbegrenzte Abgabe von Sensorimpulsen bzw. Inkrementen ermöglicht.

Der berührungssensitive Bildschirm 11 des Handbediengerätes 8 bzw. des Bedienpanels 9 ist zur Visualisierung von systemrelevanten Daten bzw. Einstellungen der Steuervorrichtung 2, 2' vorgesehen. Insbesondere sind mittels dem berührungssensitiven Bildschirm 11 steuerungstechnische Einstellungen bzw. Maschinenzustände visualisierbar und auch benutzerseitige Eingaben bzw. Einstellungsveränderungen durchführbar. Der berührungssensitive Bildschirm 11 stellt somit für eine Bedienperson 7 einerseits ein Kontrollinstrument dar, andererseits aber auch einen Teil der zuvor genannten Eingabevorrichtung 10 zur Veränderung von steuerungsrelevanten Einstellungen dar. Demzufolge ist der berührungssensitive Bildschirm 11, welcher auch als Touch-Screen bezeichnet werden kann, zur Anzeige und zur bedarfsweisen Veränderung bzw. Einstellung steuerungsrelevanter Parameter von Seiten einer Bedienperson 7 vorgesehen.

Solche steuerungsrelevanten Parameter können dabei in Bezug auf einen Industrieroboter 4 durch Bewegungsgeschwindigkeiten, durch Mindest- und/oder Maximal-Stellkräfte, durch Grenzen eines zulässigen Arbeitsbereiches, durch Einstellwerte eines vom Industrieroboter 4 geführten Werkzeuges, oder durch sonstige Maschinen-Parameter definiert sein. Die jeweiligen steuerungsrelevanten Parameter sind dabei vorzugsweise unmittelbar via den berührungssensitiven Bildschirm 11 am portablen Handbediengerät 8 bzw. am stationären Bedienpanel 9 einstell- bzw. veränderbar.

In Zusammenhang mit einer Produktionsmaschine, insbesondere in Zusammenhang mit einer in Fig. 2 veranschaulichten Spritzgießmaschine 19, können die am berührungssensitiven Bildschirm 11 des Bedienpanels 9 visualisierbaren und bedarfsweise veränderbaren, steuerungsrelevanten Parameter beispielsweise durch Soll-Temperaturen von Heizzonen, durch einen minimalen und/oder maximalen Einspritzdruck, durch ein Verhalten während einer Nachdruckfase, durch Wartezeiten zwischen einzelnen Prozessschritten, oder durch sonstige Maschinen-Parameter definiert sein. Vor allem in Verbindung mit hochautomatisierten Produktionsmaschinen, wie zum Beispiel Spritzgießmaschinen 19, gibt es typischerweise eine Vielzahl von voreinstellbaren bzw. bei Bedarf zu verändernden Parametern, um einen optimalen Prozessbzw. Fertigungsablauf erzielen zu können. Die jeweiligen Parameter werden, wie an sich bekannt, nach erfolgter Einstellung von der entsprechenden Steuervorrichtung 2, 2' übernommen bzw. im Zuge der Umsetzung des Steuerungsablaufes entsprechend verarbeitet.

Zur möglichst einfachen bzw. intuitiven und raschen Veränderung solcher Parameter durch eine Bedienperson 7 sind die nachfolgend beschriebenen technischen Maßnahmen und Bedienungsabläufe vorgesehen.

Das Drehsteller-Bedienelement 17 und der berührungssensitive Bildschirm 11 sind mit einer elektronischen Auswerte- und Steuervorrichtung 20 steuerungstechnisch verbunden. Die Auswerte- und Steuervorrichtung 20 kann dabei durch eine baulich weitgehend eigenständige Einheit definiert sein. Zweckmäßigerweise ist diese Auswerte- und Steuervorrichtung 20 jedoch in die eigentliche Maschinen-Steuervorrichtung 2' und/oder in die Steuervorrichtung 2 des Bedienpanels 9 bzw. des Handbediengerätes 8 implementiert bzw. stellt die Auswerte- und Steuervorrichtung 20 typischerweise eine Teilkomponente der Steuervorrichtung 2 und/oder 2' dar. Dies vor allem deshalb, weil die entsprechenden Funktionalitäten der Auswerte- und Steuervorrichtung 20 überwiegend softwaretechnisch implementierbar sind und die jeweiligen Funktionalitäten daher auch von der eigentlichen Maschinen-Steuervorrichtung 2' und/oder von der Steuervorrichtung 2 im Bedienpanel 9 bzw. im Handbediengerät 8 übernommen bzw. erfüllt werden können. Der Einfachheit wegen wird nachfolgend primär auf die Auswerte- und Steuervorrichtung 20 Bezug genommen, obwohl - wie dargelegt - die Auswerte- und Steuervorrichtung 20 auch als Maschinen-Steuervorrichtung 2' bzw. als Steuervorrichtung 2 des Bedienpanels 9 oder des Handbediengerätes 8 verstanden werden kann. Die Auswerte- und Steuervorrichtung 20 ist somit auch als funktionales Synonym für die Steuervorrichtung 2, 2' zu verstehen.

Die Auswerte- und Steuervorrichtung 20 ist dazu eingerichtet, insbesondere softwaretechnisch derart programmiert, dass sie eine Auswahl eines Parameters seitens einer Bedienperson 7 durch Berührung eines gewünschten Parameter-Feldes 21 erkennen kann. Verschiedene Parameter-Felder 21, welche via den berührungssensitiven Bildschirm 11 visualisierbar sind, sind exemplarisch in Fig. 3 veranschaulicht. Die Auswahl des jeweiligen Parameter-Feldes 21 erfolgt vorzugsweise durch Berührung des entsprechenden Parameter-Feldes 21 mit einem Finger der Bedienperson 7.

In den diversen Parameter-Feldern 21 sind unterschiedliche Parameterwerte 22 exemplarisch veranschaulicht. Anstelle von Zahlenwerten sind unter diesen Parameterwerten 22 auch Einstellungsstufen, wie zum Beispiel niedrig, mittel, hoch und dergleichen zu verstehen. Unter einem Parameterwert 22 sind aber auch Einstellungsoptionen bzw. Menüoptionen zu verstehen, welche nach Auswahl eines Parameter-Feldes 21 listen- bzw. tabellenartig aufscheinen und von einer Bedienperson 7 selektier- bzw. anwählbar sind.

Wesentlich ist, dass zur Einstellung bzw. Veränderung eines Parameterwertes 22 in dem von der Bedienperson ausgewählten bzw. gewünschten Parameter-Feld 21 das Betätigungsorgan 18 des Drehsteller-Bedienelementes 17 von der Bedienperson 7 um einen entsprechenden Drehwinkel zu verdrehen ist. Die Auswerte- und Steuervorrichtung 20 erfasst dabei das Ausmaß der Drehbewegung des Betätigungsorgans 18 und verändert den ausgewählten Parameterwert 22 proportional zur erfassten Drehbewegung des Betätigungsorganes 18.

Demnach ist eine Kombination in Bezug auf eine Auswahl des gewünschten Parameter-Feldes 21 durch Berührung des jeweiligen Parameter-Feldes 21 vorgesehen und nachfolgend eine Veränderung von dessen Parameterwert 22 durch einfache Verdrehung des hardwaremäßig bzw. baulich implementierten Drehsteller-Bedienelementes 17 bzw. von dessen endlos verdrehbaren Betätigungsorganes 18 vorgesehen. Dadurch kann eine besonders rasche und intuitive sowie fehlervermeidende Eingabe von Parameterwerten 22 erzielt werden.

Zweckmäßig ist es in diesem Zusammenhang, wenn der Finger einer Bedienperson 7 im ausgewählten Parameterfeld 21 oder zumindest in dessen Nahbereich verbleibt, während mittels dem Drehsteller-Bedienelement 17 bzw. mittels dem Betätigungsorgan 18 der jeweils gewünschte Parameterwert 22 eingestellt wird. Das heißt, dass das selektierte Parameter-Feld 21 seitens der Bedienperson 7 solange berührt wird, solange eine Einstellung bzw. Veränderung des entsprechenden Parameterwertes 22 vorgesehen bzw. gewünscht ist. Während einer Veränderung des jeweiligen Parameterwertes 22 liegt also eine Berührung des entsprechenden Parameter-Feldes 21 vor, und erfolgt gleichzeitig eine Drehbetätigung des Betätigungsorganes 18. Zweckmäßig ist es dabei, wenn mit einem Finger der ersten Hand der Bedienperson 7 das gewünschte Parameter-Feld 21 berührt wird, während mit der zweiten Hand der Bedienperson des Betätigungsorgan 18 des Drehsteller-Bedienelementes 17 betätigt wird.

Entsprechend einer zweckmäßigen Ausführungsform kann die Auswerte- und Steuervorrichtung 20 dazu eingerichtet sein, eine Bestätigung oder Übernahme eines eingestellten Parameterwertes 22 basierend auf einer Erkennung einer Beendigung der Berührung des Parameter-Feldes 21, insbesondere infolge eines Abhebens des Fingers der Bedienperson 7 vom entsprechenden Parameter-Feld 21, vorzunehmen oder einzuleiten. Diese steuerungstechnische Maßnahme ist vorteilhaft, um eine rasche und zugleich unmissverständliche Veränderung und Quittierung von Parameterwerten 22 zu erzielen.

Entsprechend einer weiteren Ausführungsform kann die Auswerte- und Steuervorrichtung 20 dazu eingerichtet sein, einen Abbruch eines Einstellvorganges am ausgewählten Parameterwert 22 basierend auf einer Erkennung einer streichenden Wegbewegung des am berührungssensitiven Bildschirms 11 abgestützten Fingers der Bedienperson 7 aus dem Anzeigebereich des Parameter-Feldes 21 vorzunehmen oder einzuleiten. Demnach wird bei einer Distanzierungsbewegung des Fingers der Bedienperson 7 gegenüber dem zuvor angewählten Parameter-Feld 21 die Veränderung bzw. Übernahme des geänderten Parameterwertes 22 abgebrochen. Im Zuge einer solchen Distanzierungsbewegung des Fingers erfolgt dabei eine streichende Fingerbewegung ausgehend vom Parameter-Feld 21 in umliegenden Bereiche des Parameter-Feldes 21, insbesondere in den Umgebungsbereich des ursprünglich angewählten Parameter-Feldes 21. Dadurch ist für die Auswerte- und Steuervorrichtung 20 eindeutig erkennbar, dass seitens der Bedienperson 7 ein Abbruch des Einstellvorganges gewünscht ist und ein allenfalls veränderter Parameterwert 22 nicht übernommen werden soll. Diese Vorgangsweise stellt eine praktikable und relativ intuitive Maßnahme in Verbindung mit einem gewünschten Abbruch eines Einstellvorganges dar.

Zweckmäßig kann es auch sein, wenn die Auswerte- und Steuervorrichtung 20 dazu eingerichtet ist, eine gleichzeitige Berührung des berührungssensitiven Bildschirms 11 mit mehreren Fingern zu erkennen. Insbesondere weist dabei der berührungssensitive Bildschirm 11 bzw. die Auswerte- und Steuervorrichtung 20 eine sogenannte Multi-Touch-Funktionalität auf. Wird in diesem Zusammenhang von der Auswerte- und Steuervorrichtung 20 erkannt, dass von der Bedienperson 7 mehrere Parameter-Felder 21 gleichzeitig berührt werden, insbesondere durch Verwendung mehrerer Finger, so kann vorgesehen sein, dass die jeweiligen Parameterwerte 22 der jeweiligen Parameter-Felder 21 gleichzeitig verändert werden. Dadurch kann eine besonders rasche Einstellung bzw. Veränderung mehrerer Parameter-Felder 21, welche beispielsweise Temperatur-Einstellfelder sein können, erzielt werden.

Gemäß einer weiteren Ausführung kann die Auswerte- und Steuervorrichtung 20 dazu eingerichtet sein, eine Geschwindigkeit der Wertveränderung oder eine Höhe von Wertesprüngen des Parameterwertes 22 basierend auf einer Erkennung einer Bewegung eines Fingers einer Bedienperson 7 am berührungssensitiven Bildschirm 11 nach links oder rechts, oder alternativ nach oben oder unten, vorzunehmen oder einzuleiten. Auch dadurch kann ein rascher und für eine Bedienperson 7 gut trainierbarer Einstellprozess erzielt werden.

Der entsprechende Einstellprozess kann von einer Bedienperson 7 besonders leicht nachvollzogen bzw. langfristig im Gedächtnis bleiben, wenn im Zuge einer Streichbewegung des Fingers nach links in Richtung einer 10er- oder 100er-Stelle des Parameterwertes 22, oder alternativ eine Streichbewegung des Fingers nach oben, eine schnellere oder sprunghaftere Erhöhung eines einzustellenden Parameterwertes 22 bewirkt wird. Analog dazu kann vorgesehen sein, dass im Zuge einer Streichbewegung des Fingers der Bedienperson 7 nach rechts, also in Richtung einer Einer- oder Kommastelle eines Parameterwertes 22, oder alternativ eine Streichbewegung des Fingers der Bedienperson 7 nach unten, eine langsamere oder weniger sprunghafte Absenkung eines einzustellenden Parameterwertes 22 bewirkt wird. Dabei kann auch vorgesehen sein, dass eine allfällige Streichbewegung des Fingers über die Einer- bzw. Kommastelle des Parameterwertes 22 hinaus, als Steuerbefehl zur Veränderung der Absenkgeschwindigkeit des Parameterwertes 22 verstanden wird. Entsprechendes ist denkbar, wenn die Streichbewegung des Fingers der Bedienperson 7 über die höherwertigste Stelle des Parameterwertes 22 hinausreicht. Durch diese Maßnahmen ist auch bei relativ kleinformatigen Parameter-Feldern 21 eine mühelose Veränderung der jeweiligen Aufskalierungs- bzw. Abskalierungsfaktors möglich.

Wie am besten aus Fig. 4 ersichtlich ist, kann das Betätigungsorgan 18 des Drehsteller-Bedienelementes 17 mit einem gesteuert veränderlichen Drehwiderstand-Generierungsmittel 23 in mechanischer Verbindung bzw. Wechselwirkung stehen. Insbesondere ist dabei die Drehbeweglichkeit des Betätigungsorganes 18 gesteuert hemmbar bzw. blockierbar, indem das Drehwiderstand-Generierungsmittel 23 mit dem Betätigungsorgan 18 bzw. mit dessen Drehachse 24 bewegungsgekoppelt ist bzw. in Bewegungskopplung versetzbar ist. Dabei besteht zwischen dem Drehwiderstand-Generierungsmittel 23 und dem Betätigungsorgan 18 entweder eine unmittelbare oder eine indirekte Wechselwirkung hinsichtlich der Drehbeweglichkeit bzw. hinsichtlich des Drehwiderstandes des Betätigungsorganes 18. Das steuerbare Drehwiderstand-Generierungsmittel 23 ist dabei ausgehend von der Auswerte- und Steuervorrichtung 20 ansteuerbar.

Demnach ermöglicht das Drehwiderstand-Generierungsmittel 23 eine Beaufschlagung des Drehsteller-Bedienelementes 17 bzw. von dessen Betätigungsorgan 18 mit einem bestimmten, gesteuert variablen Drehwiderstand bzw. Bremsmoment. Dieser Drehwiderstand kann dabei in Abhängigkeit der Ausführung des Drehwiderstand-Generierungsmittels 23 innerhalb mechanischer und technischer Grenzen beliebig variiert bzw. bedarfsabhängig gesteuert werden. Insbesondere können dabei Drehwiderstande bzw. Bremsmomente zwischen 0 und einem Maximalwert erzeugt werden, wobei der Maximalwert zumindest annähernd einer Blockade bzw. Sperre der Drehbeweglichkeit des Betätigungsorganes 18 entsprechen kann.

Das Drehwiderstand-Generierungsmittel 23 kann dabei durch beliebige aus dem Stand der Technik bekannte Prinzipien bzw. Systeme verwirklicht sein. Zweckmäßig ist es, wenn das Drehwiderstand-Generierungsmittel 23 Stellelemente umfasst, welche auf dem magnetorheologischen Prinzip basieren, insbesondere magnetorheologische Flüssigkeiten umfassen. Gleichsam ist es möglich, dass das Drehwiderstand-Generierungsmittel 23 mechanische bzw. elektromechanisch steuerbare bzw. aktivierbare Brems- bzw. Blockiermittel umfasst.

Entsprechend einer vorteilhaften Ausführungsform sind die Auswerte- und Steuervorrichtung 20 und das Drehwiderstand-Generierungsmittel 23 dazu eingerichtet, die Höhe des Drehwiderstandes bzw. Bremsmomentes des Betätigungsorganes 18 in Abhängigkeit eines Ausmaßes von Veränderungen des Parameterwertes 22 pro Drehwinkel des Betätigungsorganes 18 zu verändern. Insbesondere entsteht dadurch eine Beziehung bzw. Wechselwirkung zwischen der jeweiligen Veränderungshöhe bzw. der Sprunghaftigkeit der Veränderungen des Parameterwertes 22 und dem Drehwiderstand des Betätigungsorganes 18. Dadurch kann der Bedienperson 7 haptisch signalisiert werden, dass bereits weitreichende Veränderungen des Parameterwertes 22 erfolgt sind. Für eine weitere Veränderung des Parameterwertes 22 muss dabei von der Bedienperson 7 eine zunehmende Betätigungskraft aufgebracht werden. Ungewollt hohe Veränderungen von Parameterwerten 22 können dadurch vermieden bzw. hintangehalten werden.

Entsprechend einer Weiterbildung kann vorgesehen sein, dass die Auswerte- und Steuervorrichtung 20 und das Drehwiderstand-Generierungsmittel 23 den Drehwiderstand des Betätigungsorganes 18 bei großen Veränderungsschritten des Parameterwertes 22 pro Drehwinkel des Betätigungsorgans 18 erhöhen und bei vergleichsweise kleinen Veränderungsschritten des Parameterwertes 22 pro Drehwinkel des Betätigungsorganes 18 senken. Dadurch werden der Bedienperson 7 haptische Signalisierungen in Bezug auf Anhebungen oder Absenkungen eines Parameterwertes 22 bereitgestellt.

Entsprechend einer zweckmäßigen Maßnahme können die Auswerte- und Steuervorrichtung 20 und das Drehwiderstand-Generierungsmittel 23 auch dazu eingerichtet sein, den Drehwiderstand des Betätigungsorganes 18 stark anzuheben oder die Drehbeweglichkeit des Betätigungsorgans 18 zu hemmen oder zu blockieren, wenn vordefinierte Wertegrenzen des jeweiligen Parameter-Feldes 21 erreicht sind. Dadurch ist für eine Bedienperson 7 intuitiv erkennbar, dass technische Grenzen erreicht sind bzw. dass aufgrund diverser Maschinenzustände eine weitere Veränderung, insbesondere eine Anhebung oder Absenkung des ausgewählten Parameterwertes 22 nicht zulässig ist. Auch dadurch kann der Bedienkomfort, die Maschinen- bzw. Betriebssicherheit, und die Intuitivität der Mensch-Maschine-Schnittstelle 6 gesteigert werden.

Die Auswerte- und Steuervorrichtung 20 und das Drehwiderstand-Generierungsmittel 23 können auch dazu eingerichtet sein, vordefinierte Zwischenpositionen, beispielsweise 25 %, 50 % und 75 %, eines Maximalwertes oder eines Wertebereiches eines Parameter-Feldes 21 durch kurzzeitige bzw. impulsähnliche Erhöhung des Drehwiderstandes des Betätigungsorgans 18 haptisch zu signalisieren. Dadurch kann von der Bedienperson 7 gewissermaßen erfühlt werden, wie hoch das Ausmaß der gerade durchgeführten Werteveränderung ist. Einstellvorgänge können dadurch gezielter und vergleichsweise fehlerfrei ausgeführt werden.

Ferner können die Auswerte- und Steuervorrichtung 20 und das Drehwiderstand-Generierungsmittel 23 dazu eingerichtet sein, eine Vielzahl vordefinierter Raststufen in Bezug auf den Drehbetätigungsweg des Betätigungsorganes 18 zu erzeugen. Diese zahlreichen, vorzugsweise gleichmäßig über den Umfang des Betätigungsorganes verteilten Raststufen erleichtern die Einstellung von Parameterwerten 22. Insbesondere können in Bezug auf eine volle Umdrehung (360°) des Betätigungsorganes 18 zahlreiche gleichmäßig verteilte Raststufen mittels dem Drehwiderstand-Generierungsmittel 23 derart erzeugt werden, dass im Zuge einer Drehbetätigung des Betätigungsorgans 18 von einer Bedienperson 7 diese Raststufen haptisch wahrnehmbar sind. Dadurch wird erreicht, dass die Bedienperson 7 den zurückgelegten Drehwinkel des Betätigungsorganes 18 quasi spürt. Dadurch kann die Werteeinstellung in den jeweiligen Parameter-Feldern 21 besonders feinfühlig bzw. zielgenau und rasch vorgenommen werden.

Die Anzahl an solchen leicht überwindbaren jedoch fühlbaren Raststufen kann dabei fix vordefiniert sein, oder in Abhängigkeit der jeweiligen Eingabefunktion bzw. des jeweiligen Parameter-Feldes 21 automatisch angepasst sein. Je nach Erfordernis können dabei grobe oder feine Rastabstufungen vorgesehen sein, wobei bis zu 100 gleichmäßig verteilte Raststufen pro ganzer Umdrehung des Betätigungsorganes 18 von einer Bedienperson 7 ohne Mühe taktil wahrgenommen werden können.

Die zuvor angegebenen technischen Maßnahmen bzw. Verfahrensabläufe sind durch eine Kombination aus hardwaretechnischen und softwaretechnischen Komponenten umsetzbar. Das Schutzbegehren der Anmelderin richtet sich daher sowohl auf Vorrichtungsansprüche, als auch auf entsprechende Verfahrensansprüche.

Die Ausführungsbeispiele zeigen mögliche Ausführungsvarianten, wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt.

Der Schutzbereich ist durch die Ansprüche bestimmt. Die Beschreibung und die Zeichnungen sind jedoch zur Auslegung der Ansprüche heranzuziehen. Einzelmerkmale oder Merkmalskombinationen aus den gezeigten und beschriebenen unterschiedlichen Ausführungsbeispielen können für sich eigenständige erfinderische Lösungen darstellen. Die den eigenständigen erfinderischen Lösungen zugrundeliegende Aufgabe kann der Beschreibung entnommen werden.

Der Ordnung halber sei abschließend darauf hingewiesen, dass zum besseren Verständnis des Aufbaus Elemente teilweise unmaßstäblich und/oder vergrößert und/oder verkleinert dargestellt wurden.

### Bezugszeichenaufstellung

- 1: Steuerungssystem
- 2, 2': Steuervorrichtung
- 3: Maschine
- 4: Industrieroboter
- 5: Leitrechner
- 6: Mensch-Maschine-Schnittstelle
- 7: Bedienperson
- 8: Handbediengerät
- 9: Bedienpanel
- 10: Eingabevorrichtung
- 11: berührungssensitiver Bildschirm
- 12: Eingabetaste
- 13: Bewegungsantrieb
- 14: Motor
- 15: Magnetventil
- 16: Bedienelement
- 17: Drehsteller-Bedienelement
- 18: Betätigungsorgan
- 19: Spritzgießmaschine
- 20: Auswerte- und Steuervorrichtung
- 21: Parameter-Feld
- 22: Parameterwert
- 23: Drehwiderstand-Generierungsmittel
- 24: Drehachse

## Patentansprüche

1. Steuervorrichtung (2, 2') für industrielle Maschinen mit gesteuerten Bewegungsantrieben (13) für Maschinenkomponenten, umfassend
eine Mensch-Maschine-Schnittstelle (6) mit wenigstens einem Schalt- oder Bedienelement zur manuellen Beeinflussung oder Vorgabe von Verstellbewegungen von zumindest einer der Maschinenkomponenten, sowie
mit einem berührungssensitiven Bildschirm (11) zur Anzeige und bedarfsweisen Veränderung steuerungsrelevanter Parameter durch eine Bedienperson, wobei
zumindest ein Bedienelement (16) als Drehsteller-Bedienelement (17) mit einem endlos verdrehbaren Betätigungsorgan (18) ausgeführt ist, und wobei
das Drehsteller-Bedienelement (17) und der berührungssensitive Bildschirm (11) mit einer elektronischen Auswerte- und Steuervorrichtung (20) steuerungstechnisch verbunden sind,
**dadurch gekennzeichnet, dass**
die Auswerte- und Steuervorrichtung (20) dazu eingerichtet ist, eine Auswahl eines Parameters seitens einer Bedienperson durch eine stetige Berührung eines gewünschten Parameter-Feldes (21), insbesondere durch selektive Berührung mittels einem Finger, zu erkennen, und dass
zur Veränderung von Inhalten des ausgewählten Parameter-Feldes (21) oder zur Einstellung eines gewünschten Parameterwertes (22) für das ausgewählte Parameter-Feld (21) das Betätigungsorgan (18) des Drehsteller-Betätigungselementes (17) von einer Bedienperson um einen entsprechenden Drehwinkel verdreht wird, und dass dabei die Auswerte- und Steuervorrichtung (20) das Ausmaß der Drehbewegung erfasst und zur proportionalen Veränderung der Inhalte des Parameter-Feldes (21) oder des Parameterwertes (22) eingerichtet ist, und dass die Auswerte- und Steuervorrichtung (20) dazu eingerichtet ist, eine Bestätigung oder Übernahme eines eingestellten Parameterwertes (22) basierend auf einer Erkennung einer Beendigung der Berührung des Parameter-Feldes (21), insbesondere infolge eines Abhebens des Fingers der Bedienperson vom entsprechenden Parameter-Feld (21), vorzunehmen oder einzuleiten.

2. Steuervorrichtung nach Anspruch 1, **dadurch gekennzeichnet, dass** die Auswerte- und Steuervorrichtung (20) dazu eingerichtet ist, einen Abbruch eines Einstellvorganges am ausgewählten Parameterwert (22) basierend auf einer Erkennung einer streichenden Wegbewegung, insbesondere einer Distanzierungsbewegung, des am berührungssensitiven Bildschirm (11) abgestützten Fingers der Bedienperson aus einem Anzeigebereich des Parameter-Feldes (21) vorzunehmen oder einzuleiten.

3. Steuervorrichtung nach einem der vorhergehenden Ansprüche, **dadurch gekennzeichnet, dass** die Auswerte- und Steuervorrichtung (20) dazu eingerichtet ist, eine gleichzeitige Berührung des berührungssensitiven Bildschirms (11) mit mehreren Fingern zu erkennen und beim Berühren mehrerer Parameter-Felder (21) die Parameterwerte (22) der jeweiligen Parameter-Felder (21) gleichzeitig zu ändern.

4. Steuervorrichtung nach einem der vorhergehenden Ansprüche, **dadurch gekennzeichnet, dass** die Auswerte- und Steuervorrichtung (20) dazu eingerichtet ist, eine Geschwindigkeit der Wertveränderung oder eine Höhe von Wertesprüngen des Parameterwertes (22) basierend auf einer Erkennung einer Bewegung des Fingers der Bedienperson am berührungssensitiven Bildschirm (11) nach links oder rechts, oder alternativ nach oben oder unten, vorzunehmen oder einzuleiten.

5. Steuervorrichtung nach Anspruch 4, **dadurch gekennzeichnet, dass** eine Streichbewegung des Fingers nach links in Richtung einer 10er- oder 100er-Stelle des Parameterwertes (22), oder alternativ eine Streichbewegung des Fingers nach oben, eine schnellere oder sprunghaftere Erhöhung eines einzustellenden Parameterwertes (22) bewirkt.

6. Steuervorrichtung nach Anspruch 4, **dadurch gekennzeichnet, dass** eine Streichbewegung des Fingers nach rechts, also in Richtung einer Einer- oder Kommastelle eines Parameterwertes (22), oder alternativ eine Streichbewegung des Fingers nach unten, eine langsamere oder weniger sprunghafte Absenkung eines einzustellenden Parameterwertes (22) bewirkt.

7. Steuervorrichtung nach einem der vorhergehenden Ansprüche, **dadurch gekennzeichnet, dass** das Betätigungsorgan (18) mit einem gesteuert veränderlichen Drehwiderstand-Generierungsmittel (23) in mechanischer Wechselwirkung steht, und dass das Drehwiderstand-Generierungsmittel (23) von der Auswerte- und Steuervorrichtung (20) ansteuerbar ist.

8. Steuervorrichtung nach Anspruch 7, **dadurch gekennzeichnet, dass** die Auswerte- und Steuervorrichtung (20) und das Drehwiderstand-Generierungsmittel (23) dazu eingerichtet sind, die Höhe des Drehwiderstandes des Betätigungsorganes (18) in Abhängigkeit eines Ausmaßes von Veränderungen, insbesondere in Abhängigkeit von unterschiedlichen Veränderungshöhen oder Veränderungssprüngen des Parameterwertes (22) pro Drehwinkel des Betätigungsorganes (18) zu verändern.

9. Steuervorrichtung nach Anspruch 8, **dadurch gekennzeichnet, dass** die Auswerte- und Steuervorrichtung (20) und das Drehwiderstand-Generierungsmittel (23) dazu eingerichtet sind, den Drehwiderstand des Betätigungsorganes (18) bei großen Veränderungsschritten des Parameterwertes (22) pro Drehwinkel des Betätigungsorganes (18) zu erhöhen und bei vergleichsweise kleinen Veränderungsschritten des Parameterwertes (22) pro Drehwinkel des Betätigungsorganes (18) zu senken.

10. Steuervorrichtung nach Anspruch 7, **dadurch gekennzeichnet, dass** die Auswerte- und Steuervorrichtung (20) und das Drehwiderstand-Generierungsmittel (23) dazu eingerichtet sind, den Drehwiderstand des Betätigungsorganes (18) stark anzuheben, oder die Drehbeweglichkeit des Betätigungsorganes (18) zu hemmen oder zu blockieren, wenn vordefinierte Wertegrenzen des jeweiligen Parameter-Feldes (21) erreicht sind.

11. Steuervorrichtung nach Anspruch 7, **dadurch gekennzeichnet, dass** die Auswerte- und Steuervorrichtung (20) und das Drehwiderstand-Generierungsmittel (23) dazu eingerichtet sind, vordefinierte Zwischenpositionen, beispielsweise 25%, 50% und 75%, eines Maximalwertes oder eines Wertebereiches eines Parameter-Feldes (21) durch kurzzeitige Erhöhung des Drehwiderstandes des Betätigungsorganes (18) haptisch zu signalisieren.

12. Steuervorrichtung nach Anspruch 7, **dadurch gekennzeichnet, dass** die Auswerte- und Steuervorrichtung (20) und das Drehwiderstand-Generierungsmittel (23) dazu eingerichtet sind, in Abhängigkeit des Vorliegens von großen oder kleinen Veränderungsschritten eines Parameterwertes (22) eine unterschiedliche Anzahl von Raststufen zu erzeugen, welche Raststufen über eine volle Umdrehung des Betätigungsorganes (18) gleichmäßig verteilt sind und im Zuge einer Drehbetätigung des Betätigungsorgans (18) von einer Bedienperson haptisch wahrnehmbar sind.

13. Verfahren zum Betreiben einer Steuervorrichtung (2, 2') für industrielle Maschinen mit gesteuerten Bewegungsantrieben (13) für Maschinenkomponenten, umfassend
eine Mensch-Maschine-Schnittstelle (6) mit wenigstens einem Bedienelement (16) zur manuellen Beeinflussung oder Vorgabe von Verstellbewegungen von zumindest einer der Maschinenkomponenten, sowie
mit einem berührungssensitiven Bildschirm (11) zur Anzeige und bedarfsweisen Veränderung steuerungsrelevanter Parameter durch eine Bedienperson, wobei
zumindest ein Bedienelement (16) als Drehsteller-Bedienelement (17) mit einem endlos verdrehbaren Betätigungsorgan (18) ausgeführt ist, und
das Drehsteller-Bedienelement (17) und der berührungssensitive Bildschirm (11) mit einer elektronischen Auswerte- und Steuervorrichtung (20) steuerungstechnisch verbunden sind,
**dadurch gekennzeichnet, dass**
die Auswerte- und Steuervorrichtung (20) dazu eingerichtet ist, eine Auswahl eines Parameters seitens einer Bedienperson durch eine stetige Berührung eines gewünschten Parameter-Feldes (21), insbesondere durch selektive Berührung mittels einem Finger, zu erkennen, und dass
zur Veränderung von Inhalten des ausgewählten Parameter-Feldes (21) oder zur Einstellung eines gewünschten Parameterwertes (22) für das ausgewählte Parameter-Feld (21) das Betätigungsorgan (18) des Drehsteller-Betätigungselementes (17) von einer Bedienperson um einen entsprechenden Drehwinkel verdreht wird, und dass dabei das Ausmaß der Drehbewegung von der Auswerte- und Steuervorrichtung (20) erfasst und zur proportionalen Veränderung der Inhalte des Parameter-Feldes (21) oder des Parameterwertes (22) verwendet wird, und dass
von der Auswerte- und Steuervorrichtung (20) eine Bestätigung oder Übernahme eines eingestellten Parameterwertes (22) basierend auf einer Erkennung einer Beendigung der Berührung des Parameter-Feldes (21), insbesondere infolge eines Abhebens des Fingers der Bedienperson vom entsprechenden Parameter-Feld (21), vorgenommen oder eingeleitet wird.

## Claims

1. A control device (2, 2') for industrial machines with controlled movement drives (13) for machine components, comprising
a human-machine-interface (6) having at least one switching or operating element for manually influencing or predetermining adjustment movements of at least one of the machine components, and
having a touch-sensitive screen (11) for displaying and optionally changing control-relevant parameters by an operator, wherein
at least one operating element (16) is formed as a rotary adjuster operating element (17) with an endlessly rotatable actuation member (18), and wherein
the rotary adjuster operating element (17) and the touch-sensitive screen (11) are connected with an electronic evaluation and control device (20) in a control-based manner,
**characterized in that**
the evaluation and control device (20) is configured to recognize a selection of a parameter on the part of an operator by means of a steady touch of a desired parameter field (21), in particular by a selective touch by a finger, and that
in order to change contents of the selected parameter field (21) or to set a desired parameter value (22) for the selected parameter field (21), the actuation member (18) of the rotary adjuster actuation element (17) is turned by an appropriate rotation angle by an operator, and that, in doing so, the evaluation and control device (20) detects the extent of the rotation movement and is configured for proportionally changing the contents of the parameter field (21) or of the parameter value (22), and that
the evaluation and control device (20) is configured to carry out or initiate a confirmation or acceptance of a set parameter value (22) based on a recognition of a termination of the touch to the parameter field (21), in particular following a lifting of the finger of the operator off the respective parameter field (21).

2. The control device according to claim 1, **characterized in that** the evaluation and control device (20) is configured to carry out or initiate a termination of a setting operation on the selected parameter value (22) based on a recognition of a swiping movement, in particular a distancing movement, of the finger of the operator resting on the touch-sensitive screen (11) out of the display region of the parameter field (21).

3. The control device according to one of the preceding claims, **characterized in that** the evaluation and control device (20) is configured to recognize a simultaneous touch of the touch-sensitive screen (11) by multiple fingers, and upon multiple parameter fields (21) being touched, to change the parameter values (22) of the respective parameter fields (21) simultaneously.

4. The control device according to one of the preceding claims, **characterized in that** the evaluation and control device (20) is configured to carry out or initiate a speed of the value changes or a level of value jumps of the parameter value (22) based on a recognition of a movement of the finger of the operator on the touch-sensitive screen (11) to the left or right, or alternatively, up or down.

5. The control device according to claim 4, **characterized in that** a swiping movement of the finger to the left in the direction of a tens digit or hundreds digit of the parameter value (22), or alternatively an upward swiping movement of the finger, causes a faster or abrupt increase of the parameter value (22) to be set.

6. The control device according to claim 4, **characterized in that** a swiping movement of the finger to the right, i. e. in the direction of a ones digit or decimal point of a parameter value (22), or alternatively a downwards swiping movement of the finger, causes a slower or less abrupt decrease of a parameter value (22) to be set.

7. The control device according to one of the preceding claims, **characterized in that** the actuation member (18) mechanically interacts with a rotational resistance-generating means (23) that can be changed in controlled manner, and that the rotational resistance-generating means (23) can be triggered by the evaluation and control device (20).

8. The control device according to claim 7, **characterized in that** the evaluation and control device (20) and the rotational resistance-generating means (23) are configured to change the level of the rotational resistance of the actuation member (18) depending on an extent of changes, in particular depending on different change levels or change jumps of the parameter value (22) per rotation angle of the actuation member (18).

9. The control device according to claim 8, **characterized in that** the evaluation and control device (20) and the rotational resistance-generating means (23) are configured to increase the level of the rotational resistance of the actuation member (18) in the event of great steps of change of the parameter value (22) per rotation angle of the actuation member (18) and to decrease it in the event of comparatively small steps of change of the parameter value (22) per rotation angle of the actuation member (18).

10. The control device according to claim 7, **characterized in that** the evaluation and control device (20) and the rotational resistance-generating means (23) are configured to greatly increase the rotational resistance of the actuation member (18), or to inhibit or block the rotatability of the actuation member (18) if predefined value limits of the respective parameter field (21) are reached.

11. The control device according to claim 7, **characterized in that** the evaluation and control device (20) and the rotational resistance-generating means (23) are configured to haptically signal intermediate positions, for example 25 %, 50 % and 75 %, of a maximum value or a value region of a parameter field (21) by means of a temporary increase of the rotational resistance of the actuation member (18).

12. The control device according to claim 7, **characterized in that** the evaluation and control device (20) and the rotational resistance-generating means (23) are configured to create a different number of locking steps depending on the presence of great or small steps of change of a parameter value (22), which locking steps are evenly distributed around a full revolution of the actuation member (18) and are haptically noticeable in the course of a rotation actuation of the actuation member (18) by an operator.

13. A method for operating a control device (2, 2') for industrial machines with controlled movement drives (13) for machine components, comprising
a human-machine-interface (6) having at least one operating element (16) for manually influencing or predetermining adjustment movements of at least one of the machine components, and
having a touch-sensitive screen (11) for displaying and optionally changing control-relevant parameters by an operator, wherein
at least one operating element (16) is formed as a rotary adjuster operating element (17) with an endlessly rotatable actuation member (18), and
the rotary adjuster operating element (17) and the touch-sensitive screen (11) are connected with an electronic evaluation and control device (20) in a control-based manner,
**characterized in that**
the evaluation and control device (20) is configured to recognize a selection of a parameter on the part of an operator by means of a steady touch of a desired parameter field (21), in particular by a selective touch by a finger, and that in order to change contents of the selected parameter field (21) or to set a desired parameter value (22) for the selected parameter field (21), the actuation member (18) of the rotary adjuster actuation element (17) is turned by an appropriate rotation angle by an operator, and that, in doing so, the extent of the rotation movement is detected and used for proportionally changing the contents of the parameter field (21) or of the parameter value (22) by the evaluation and control device (20), and that a confirmation or acceptance of a set parameter value (22) based on a recognition of a termination of the touch to the parameter field (21), in particular following a lifting of the finger of the operator off the respective parameter field (21) is carried out or initiated by the evaluation and control device (20).

## Revendications

1. Dispositif de commande (2, 2') pour des machines industrielles avec des entraînements de mouvement (13) contrôlés pour des composants de machines, comprenant
une interface homme-machine (6) avec au moins un élément de commutation ou de commande pour le contrôle manuel ou l'imposition de mouvements de réglage d'au moins un des composants de la machine ainsi que
avec un écran tactile (11) pour l'affichage et la modification si nécessaire de paramètres concernant le contrôle par un opérateur, dans lequel
au moins un élément de commande (16) est conçu comme un élément de commande à actionneur rotatif (17) avec un organe d'actionnement rotatif sans fin (18) et dans lequel l'élément de commande à actionneur rotatif (17) et l'écran tactile (11) sont reliés, à des fins de contrôle, avec un dispositif électronique d'analyse et de commande (20), **caractérisé en ce que**
le dispositif d'analyse et de commande (20) est conçu pour détecter une sélection d'un paramètre par un opérateur par un contact prolongé avec un champ de paramètre souhaité (21), plus particulièrement par le contact sélectif au moyen d'un doigt et **en ce que**
pour la modification de contenus du champ de paramètre sélectionné (21) ou pour le réglage d'une valeur de paramètre souhaitée (22) pour le champ de paramètre sélectionné (21), l'organe d'actionnement (18) de l'élément d'actionnement à actionneur rotatif (17) est tourné par un opérateur d'un angle de rotation correspondant et **en ce que** le dispositif d'analyse et de commande (20) détecte l'amplitude du mouvement de rotation et est conçu pour une modification proportionnelle des contenus du champ de paramètre (21) ou de la valeur de paramètre (22) et **en ce que** le dispositif d'analyse et de commande (20) est conçu pour effectuer ou initier une confirmation ou une validation d'une valeur de paramètre réglée (22) sur la base d'une détection d'une fin du contact avec le champ de paramètre (21), plus particulièrement à la suite d'un relâchement du contact du doigt de l'opérateur avec le champ de paramètre (21) correspondant.

2. Dispositif de commande selon la revendication 1, **caractérisé en ce que** le dispositif d'analyse et de commande (20) est conçu pour effectuer ou initier une interruption d'un processus de réglage sur la valeur de paramètre sélectionnée (22) sur la base d'une détection d'un mouvement d'effleurement, plus particulièrement d'un mouvement de distanciation du doigt de l'opérateur appuyé sur l'écran tactile (11), hors d'une zone d'affichage du champ de paramètre (21).

3. Dispositif de commande selon l'une des revendications précédentes, **caractérisé en ce que** le dispositif d'analyse et de commande (20) est conçu pour détecter un contact simultané de l'écran tactile (11) avec plusieurs doigts et, lors du contact avec plusieurs champs de paramètres (21), pour modifier simultanément les valeurs de paramètres (22) des champs de paramètres (21) respectifs.

4. Dispositif de commande selon l'une des revendications précédentes, **caractérisé en ce que** le dispositif d'analyse et de commande (20) pour effectuer ou initier une vitesse de la modification des valeurs ou une amplitude des sauts de valeurs de la valeur de paramètre (22) sur la base d'une détection d'un mouvement du doigt de l'opérateur sur l'écran tactile (11) de la gauche vers la droite ou en variante de haut en bas.

5. Dispositif de commande selon la revendication 4, **caractérisé en ce qu'**un mouvement d'effleurement du doigt vers la gauche en direction d'une dizaine ou d'une centaine de la valeur de paramètre (22) ou en variante un mouvement d'effleurement du doigt vers le haut, provoque une augmentation plus rapide ou plus brusque d'une valeur de paramètre (22) à régler.

6. Dispositif de commande selon la revendication 4, **caractérisé en ce qu'**un mouvement d'effleurement du doigt vers la droite, donc en direction d'une unité ou d'une virgule d'une valeur de paramètre (22) ou en variante un mouvement d'effleurement du doigt vers le bas, provoque une diminution plus lente ou moins brusque d'une valeur de paramètre (22) à régler.

7. Dispositif de commande selon l'une des revendications précédentes, **caractérisé en ce que** l'organe d'actionnement (18) est en interaction mécanique avec un moyen de génération de résistance à la rotation (23) variable de manière contrôlée et **en ce que** le moyen de génération de résistance à la rotation (23) peut être contrôlé par le dispositif d'analyse et de commande (20).

8. Dispositif de commande selon la revendication 7, **caractérisé en ce que** le dispositif d'analyse et de commande (20) et le moyen de génération de résistance à la rotation (23) sont conçus pour modifier la valeur de la résistance à la rotation de l'organe d'actionnement (18) en fonction d'une amplitude des modifications, plus particulièrement en fonction de différentes valeurs de modifications ou de sauts de modifications de la valeur du paramètre (22) par angle de rotation de l'organe d'actionnement (18).

9. Dispositif de commande selon la revendication 8, **caractérisé en ce que** le dispositif d'analyse et de commande (20) et le moyen de génération de résistance à la rotation (23) sont conçus pour augmenter la résistance à la rotation de l'organe d'actionnement (18) avec de grands pas de modification de la valeur de paramètre (22) par angle de rotation de l'organe d'actionnement (18) et pour la diminuer avec de petits pas de modification de la valeur de paramètre (22) par angle de rotation de l'organe d'actionnement (18).

10. Dispositif de commande selon la revendication 7, **caractérisé en ce que** le dispositif d'analyse et de commande (20) et le moyen de génération de résistance à la rotation (23) sont conçus pour augmenter fortement la résistance à la rotation de l'organe d'actionnement (18) ou pour empêcher ou pour bloquer la mobilité en rotation de l'organe d'actionnement (18) lorsque des limites de valeurs prédéfinies du champ de paramètre (21) respectif sont atteintes.

11. Dispositif de commande selon la revendication 7, **caractérisé en ce que** le dispositif d'analyse et de commande (20) et le moyen de génération de résistance à la rotation (23) sont conçus pour signaler de manière haptique des positions intermédiaires prédéfinies, par exemple 25 %, 50 % et 75 % d'une valeur maximale ou d'une plage de valeurs d'un champ de paramètre (21) par l'augmentation brève de la résistance à la rotation de l'organe d'actionnement (18).

12. Dispositif de commande selon la revendication 7, **caractérisé en ce que** le dispositif d'analyse et de commande (20) et le moyen de génération de résistance à la rotation (23) sont conçus pour générer, en fonction de la présence de grands ou de petits pas de modification d'une valeur de paramètre (22), un nombre différent de crans, ces crans étant répartis régulièrement sur un tour complet de l'organe d'actionnement (18) et étant perceptibles de manière haptique lors d'un actionnement en rotation de l'organe d'actionnement (18) par un opérateur.

13. Procédé de fonctionnement d'un dispositif de commande (2, 2') pour des machines industrielles avec des entraînements de mouvements contrôlés (13) pour des composants de machines, comprenant
une interface homme-machine (6) avec au moins un élément de commande (16) pour le contrôle ou l'imposition manuelle de mouvements de réglage d'au moins un des composants de la machine, ainsi que
avec un écran tactile (11) pour l'affichage et la modification si nécessaire de paramètres concernant le contrôle par un opérateur, dans lequel
au moins un élément de commande (16) est conçu comme un élément de commande à actionneur rotatif (17) avec un organe d'actionnement rotatif sans fin (18) et l'élément de commande à actionneur rotatif (17) et l'écran tactile (11) sont reliés, à des fins de contrôle, avec un dispositif électronique d'analyse et de commande (20), **caractérisé en ce que**
le dispositif d'analyse et de commande (20) est conçu pour détecter une sélection d'un paramètre par un opérateur par un contact prolongé avec un champ de paramètre souhaité (21), plus particulièrement par le contact sélectif au moyen d'un doigt et **en ce que**
pour la modification de contenus du champ de paramètre sélectionné (21) ou pour le réglage d'une valeur de paramètre souhaitée (22) pour le champ de paramètre sélectionné (21), l'organe d'actionnement (18) de l'élément d'actionnement à actionneur rotatif (17) est tourné par un opérateur d'un angle de rotation correspondant et **en ce que** l'amplitude du mouvement de rotation est détectée par le dispositif d'analyse et de commande (20) et est utilisée pour une modification proportionnelle des contenus du champ de paramètre (21) ou de la valeur de paramètre (22) et **en ce que** le dispositif d'analyse et de commande (20) effectue ou initie une confirmation ou une validation d'une valeur de paramètre réglée (22) sur la base d'une détection d'une fin du contact avec le champ de paramètre (21), plus particulièrement à la suite d'un relâchement du contact du doigt de l'opérateur avec le champ de paramètre (21) correspondant.
